(19) Europäisches Patentamt / European Patent Office / Office européen des brevets

(11) **EP 4 478 372 A1**

(12) **EUROPEAN PATENT APPLICATION**

(43) Date of publication:
**18.12.2024 Bulletin 2024/51**

(21) Application number: **23179491.8**

(22) Date of filing: **15.06.2023**

(51) International Patent Classification (IPC):
**G16H 20/30** (2018.01)    **G16H 20/70** (2018.01)
**G16H 10/60** (2018.01)    **A61M 21/02** (2006.01)
**A63B 23/18** (2006.01)    A61M 21/00 (2006.01)

(52) Cooperative Patent Classification (CPC):
**A61M 21/02; G16H 10/60; G16H 20/30;**
**G16H 20/70;** A61M 2021/0022; A61M 2021/0027;
A61M 2021/0044; A61M 2230/42; A63B 23/185;
A63B 71/0622; A63B 2071/0625; A63B 2071/0655;
A63B 2225/62

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventor: **JIN, Sheng**
**Eindhoven (NL)**

(74) Representative: **Philips Intellectual Property &**
**Standards**
**High Tech Campus 52**
**5656 AG Eindhoven (NL)**

(54) **PROVIDING BREATHING GUIDANCE TO AN INDIVIDUAL**

(57)    A mechanism for guiding the length of a plurality of different segments of a plurality of breathing cycles. A recorded length of each of the plurality of segments in a sample breathing cycle is obtained. The recorded length of each segment is used, together with a sample breathing rate and a desired breathing rate, to define a target length of each segment in each of a plurality of breathing cycles. Each target length is proportional to the corresponding recorded length.

FIG. 3

**Description**

FIELD OF THE INVENTION

The present invention relates to the field of breathing guidance.

BACKGROUND OF THE INVENTION

A slow and regular breathing action is considered beneficial for relaxation. More specifically, research has shown that paced or guided breathing leads to a physiological effect or outcome in humans/mammals, including reduced blood pressure and improved oxygenation of blood. One particularly useful use-case scenario for providing paced/guided breathing is to encourage or aid in falling asleep. More particularly, the physiological changes in an individual that result from paced/guided breathing are similar to those that occur whilst the individual is falling asleep.

There are a number of available breathing guidance devices that aim to encourage paced or guided breathing, including mobile phone applications or similar.

One example device is a guided breathing hugging pillow. The surface of the pillow can fluctuate up and down (e.g., by inflating/deflating an airbag) to simulate a breathing pattern for the individual. The individual can feel and follow this pattern to control their breathing by tactile sensing while touching the pillow.

There is an ongoing desire to improve the effectiveness of a breathing guidance.

SUMMARY OF THE INVENTION

The invention is defined by the claims.

According to examples in accordance with an aspect of the invention, there is provided a computer-implemented method for controlling an output device to provide paced breathing guidance to an individual.

The computer-implemented method comprises obtaining a recorded length for each of at least two different segments in a sample breathing cycle of the individual, each recorded length being the length of a respective segment in the sample breathing; obtaining a sample breathing rate of the individual; obtaining a desired breathing rate for the individual; and determining, for a further breathing cycle of the individual, for at least one of the at least two different segments, a target length by processing the recorded length for said segment, the desired breathing rate for the individual, and the sample breathing rate for the individual, wherein the target length for said segment is proportional to the recorded length for said segment; and generating a control signal for the output device to provide a user-perceptible output for guiding the individual to breathe, in accordance with the target length.

Embodiments provide a technique for paced or guided breathing, in which the length of a segment of the paced breathing is controlled to be proportional to the length of the respective segment during a sample breathing cycle (i.e., an initial breathing cycle before paced breathing is provided).

The proposed approach provides a more natural breathing cycle for the individual, as it will more closely match the rhythm of their own breathing cycle. This increases a likelihood of adherence to the paced breathing cycle, increased for an individual in following paced breathing guidance and improved outcomes for the individual undergoing paced breathing.

In some examples, for at least one of the at least two different segments, the target length is proportional to the recorded length for said segment multiplied by a ratio of the sample breathing rate and the desired breathing rate. This approach significantly increases an ease for the individual in following the paced breathing guidance, as the rhythm of the paced breathing guidance will match that of a sample breathing cycle and therefore be more representative of the individual's breathing technique.

In some examples, at least two different segments comprise at least one breath holding segment. This approach recognizes that a natural breath cycle of an individual will contain a breath hold, allowing for improved control and adherence for paced breathing guidance.

In some examples, at least one breath holding segment comprises a post-inhalation breath holding segment and a post-exhalation breath holding segment.

In at least one example, each breath holding segment, the target length is the same as the recorded length of the sample breathing cycle. This approach recognizes that holding of a breath may be difficult or disruptive to an individual undergoing paced breathing guidance. By setting the target length of a breath holding segment to be the same as that of a sample breathing cycle, this disruption is advantageously avoided whilst still facilitating a change to the breathing rate of the individual.

In some examples, for each breath holding segment, the difference between the target length and the recorded length is non-zero.

In some examples, for each breath holding segment, the target length is proportional to the recorded length for

said segment multiplied by a ratio of the sample breathing rate and the desired breathing rate.

In preferred examples, for each breath holding segment, the target length is proportional to a weight that changes responsive to the sampled breathing rate. For example, user may have longer or shorter target length for the breach holding segment when the user's breath rate is lower. Hence, a weight can be added to realize the adjustment of the target length.

In at least one embodiment, the at least two different segments comprise an inhalation segment and an exhalation segment. This facilitates control or guidance over two known segments of a breathing cycle of the individual.

In some embodiments, a ratio between the target length of the inhalation segment and the recorded length of the inhalation segment is greater than a ratio between the target length of said breath holding segment and the recorded length of the breath holding segment; and/or a ratio between the target length of the exhalation segment and the recorded length of the exhalation segment is greater than a ratio between the target length of said breath holding segment and the recorded length of the breath holding segment.

In some embodiments, a ratio between the target length of the inhalation segment and the recorded length of the inhalation segment is the same as a ratio between the target length of said breath holding segment and the recorded length of said breath holding segment; and/or a ratio between the target length of the exhalation segment and the recorded length of the exhalation segment is the same as a ratio between the target length of said breath holding segment and the recorded length of said breath holding segment.

In some examples, for the inhalation segment and the exhalation segment, the target lengths of each segment are proportional to the recorded length for said segment multiplied by a ratio of the sample breathing rate and the desired breathing rate.

In some examples, for the further breath holding segment, the target length is proportional to the recorded length for said segment multiplied by a ratio of the sample breathing rate and the desired breathing rate.

In embodiments, the method comprises obtaining a recorded length for all segments in the sample breathing cycle; determining a target length for all segments in the sample breathing cycle, wherein the sum of all target lengths, when defined in seconds, is equal to 60 times the reciprocal of the desired breathing rate, when defined in breaths per minute.

In some embodiments, the step of obtaining a desired breathing rate comprises obtaining a desired breathing rate for each segment for a of the plurality of further breathing cycles of the individual; and for each of the plurality of further breathing cycles, the step of determining a target length for each segment comprises processing the recorded length for said segment, the desired breathing rate for the breathing cycle of the individual, and the sample breathing rate for the individual, wherein the target length for said segment is proportional to the recorded length for said segment.

This technique allows for an evolution or change of the paced breathing guidance over time, for instance, to allow a gradual transition of the paced breathing towards a final target breathing rate over at least one of the plurality of further breathing cycles.

In some examples, the desired breathing rate initially starts at the sample breathing rate and gradually changes to a target breathing rate.

There is also provided a computer program product comprising computer program code means which, when executed on a computing device having a control system, cause the control system to perform all of the steps of any herein disclosed method.

There is also provided a system for providing paced breathing guidance to an individual, the system comprising an output device for providing user-perceptible outputs to an individual and a control system.

The control system is configured to obtain a recorded length for each of at least two different segments in a sample breathing cycle (200) of the individual, each recorded length being the length of a respective segment in a sample breathing cycle of the individual; obtain a sample breathing rate of the individual; obtain a desired breathing rate for the individual; and determining, for a further breathing cycle of the individual, for at least one of the at least two different segments, a target length by processing the recorded length for said segment, the sample breathing rate and the desired breathing rate for the individual, wherein the target length for said segment is proportional to the recorded length for said segment; and generate a control signal for the output device to provide a user-perceptible output for guiding the individual to breathe, for the breathing cycle, in accordance with the target length.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system in which embodiments can be employed;

Fig. 2 conceptually illustrates a breathing cycle;

Fig. 3 is a flowchart illustrating a proposed method; and

Fig. 4 illustrates a display for obtaining recorded lengths of different segments of a sample breathing cycle.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for guiding the length of a plurality of different segments of a plurality of breathing cycles. A recorded length of each of the plurality of segments in a sample breathing cycle is obtained. The recorded length of each segment is used, together with a sample breathing rate and a desired breathing rate, to define a target length of each segment in each of a plurality of breathing cycles. Each target length is proportional to the corresponding recorded length.

Embodiments are based on the recognition that different individuals will have different rhythms to their breathing cycles. Compliance or adherence to paced breathing guidance is more difficult if the rhythm of the individual's breathing does not match that of the paced breathing guidance. By controlling segments of paced breathing cycles to be proportional to recorded length(s) of said segments, an ease of following paced breathing is increased.

Proposed approaches find particular advantage in guided sleep scenarios, but can be employed in any circumstance in which paced breathing guidance is desired.

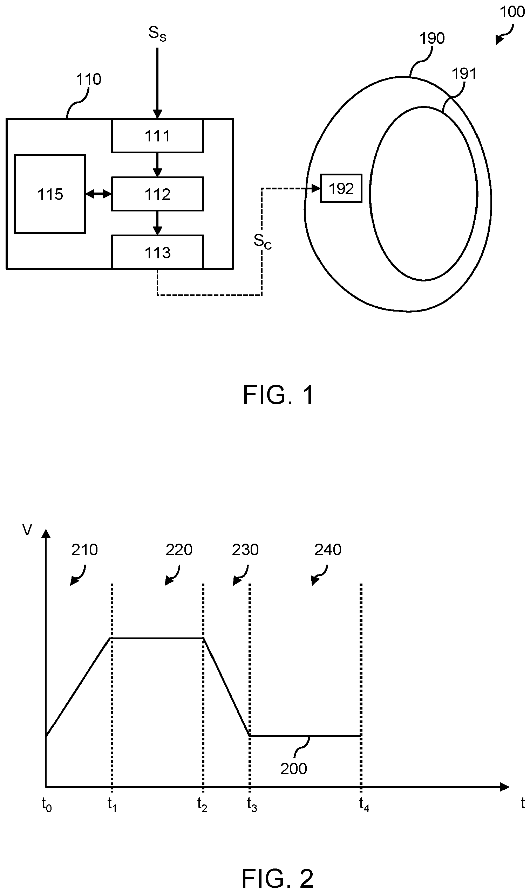

Fig. 1 illustrates a system 100 in which embodiments can be employed, for improved contextual understanding.

The system 100 comprises a control system 110 and an output device 190.

The output device 190 is here configured to controllably provide a user-perceptible output for providing paced or guided breathing to an individual. The control system 110 defines or controls the operation of the output device, as later described.

A wide variety of different types of output device 190 could be used to provide a user-perceptible output.

In one example, the output device 190 is configured to provide tactile guidance of breathing for an individual. In this way, the output device 190 is able to provide a user-perceptible output in the form of a tactile output for paced or guided breathing. In the illustrated example, the output device 190 here comprises a moveable member 191 that is able to move to guide the breathing of an individual.

The moveable member 191 can therefore be controlled to move to provide the tactile guidance to the individual, i.e., provide a tactile output to the individual. For instance, the moveable member 191 may move or fluctuate a surface of the output device according to a desired breathing rate and/or pattern, to guide the individual to follow the breathing rate and/or pattern.

One example of a moveable member 191 is an airbag with controllable inflation and deflation. Another example is a piston-based system for providing tactile feedback. Yet another example is a motor-driven paddle. Other examples will be apparent to the skilled person.

The output device may be formed as a (huggable) pillow or cushion, e.g., with a soft surface material. This is natural for an individual to hug or squeeze when preparing to sleep or when falling asleep, to ensure that the output device is able to provide the tactile feedback in a natural manner. However, this is not essential. In another example, the output device may be a hand-held device that can be squeezed or held in the hand, e.g., a soft toy, stress ball or the like.

It will be readily apparent that other suitable forms of output device for facilitating the provision of paced or guided breathing to an individual using a user-perceptible output will be apparent to the skilled person. One alternative to a moveable member is a screen configured to provide a visual output that can be used to provide paced/guided breathing (e.g., using a pulsating icon or the like). Another alternative to a moveable member is a speaker or buzzer for providing an audible output that can be used to provide paced/guided breathing. Such approaches are well known.

The output device may also comprise a device control system 192, configured to control the operation of the output device (e.g., the moveable member, screen or speaker). The device control system 192 may, for instance, comprise a microcontroller and/or other control logic for controlling the operation of output device 190.

The control system 110 is configured to control or define the operation of the output device 190.

The control system 110 here comprises an input control interface 111, a data processor 112 and an output control interface 113. The data processor 112 of the control system 110 is configured to control the operation of the output device 190 via the output control interface 113.

Accordingly, the output control interface 113 is communicatively coupled to the output device to control the operation thereof. In particular, the output control interface 113 may be communicatively coupled to the device control system 192, to control the operation of the output device using the capabilities of the device control system 192, e.g., using a control signal Sc. For instance, the control system may control the operation of the device control system 192 by providing instructions for execution by the device control system.

The communication between the output control interface 113 and the output device 190 may be wired or wireless. Suitable wireless communication protocols that may be used to communicate with the tactile breathing guidance device 190 include an infrared link, Zigbee, Bluetooth, a wireless local area network protocol such as in accordance with the IEEE 802.11 standards, a 2G, 3G, 4G or 5G telecommunication protocol, and so on. Other formats will be readily apparent to the person skilled in the art.

The control system 110 is configured to determine a target length for each of a plurality of segments of a plurality of breathing cycles. The control system 110 controls the output device to provide a user-perceptible output to breathe, for each breathing cycle, in accordance with the target length of each of the at least two different segments.

For improved contextual understanding, Fig. 2 is a Figure that illustrates a single breathing cycle 200 of an individual. An x-axis represents time t (e.g., in seconds) and a y-axis represents volume V of air (e.g., in $m^3$) in the individual's lungs.

A breathing cycle 200 can be conceptually divided into a plurality of different segments. A segment is therefore a part or portion of a breathing cycle. The plurality of segments may include at least an inhalation segment 210 (where an individual is breathing in) and an exhalation segment 230 (where the individual is breathing out).

The breathing cycle 200 may further include at least one breath holding segment 220, 240. One example of a breath holding segment may be labelled an inhalation breath holding segment 220, which takes place between an inhalation 210 and exhalation 230 segment. Another example of a breath holding segment may be labelled an exhalation breath holding segment 240, which takes place after an exhalation segment 230, and before the next breathing cycle. One or both of the inhalation and exhalation breath holding segments can be omitted in some scenarios.

It will be appreciated that there may be further segments of a breathing cycle, which are not in the illustrated example of a breathing cycle.

For instance, a breathing cycle may comprise a plurality of inhalation segments, e.g., a first inhalation segment during which a volume of air increases at a first rate, and a second inhalation segment, during which a volume of air increases at a second, different rate. As another example, a breathing cycle may comprise a plurality of temporally separated inhalation segments, e.g., separated by one or more breath holding segments and/or exhalation segments.

Similar approaches for forming a breathing cycle that comprises a plurality of exhalation segments will be apparent to the skilled person.

Thus, a breathing cycle 200 may comprise one or more inhalation segments 210, one or more exhalation segments 230 and (optionally) one or more breath holding segments 220, 240. The precise arrangement of a breathing cycle may, for instance, be dependent upon a specific individual and/or a desired paced breathing guidance for the individual, e.g., to target deep inhalation or deep exhalation for certain medical conditions or pathologies.

The present invention proposes a technique for determining a target length for a plurality of different segments of a plurality of breathing cycles. A user-perceptible output (provided by an output device) is then controlled to guide an individual to breath in accordance with the determined target lengths.

More particularly, the present disclosure provides a technique for setting a target length that makes use of recorded lengths of a sample breathing cycle of the individual. This allows the target lengths of each segment to be turned to the sample breathing cycle to more closely match the rhythm or pattern of breathing of the individual. This increases a likelihood of compliance.

Approaches for controlling an output device to provide breathing guidance in accordance with determined target lengths of different segments of breathing cycles will be readily apparent to the skilled person, and will depend upon the nature of the output device.

More particularly, the output device may be controlled to control the timings of different forms of feedback or user-perceptible outputs responsive to the target lengths of each segment. In particular, the target length of each segment may define for how long a first form of feedback or user-perceptible output is provided until said form of feedback is ended. During paced breathing guidance, the timing of different forms of feedback may be sequentially controlled in accordance with the target length of each segment.

By way of example, for an output device that having a moveable member, a movement (or non-movement) of the moveable member may be controlled to correspond to a length of a breathing segment. For instance, movement in a first direction may represent an inhalation segment, a lack of movement may represent a breath holding segment and a movement in a second (different) direction may represent an exhalation segment.

As another example, for an output device having a speaker, a pitch of a tone emitted by the speaker may be controlled to correspond to a particular segment. For instance, an increasing pitch may represent an inhalation segment, a maintained pitch may represent a breath holding segment and a decreasing pitch may represent an exhalation segment.

As yet another example, for an output device having a screen or display, an appearance of an icon may be modified responsive to a segment. For instance, the icon may be: expanded in size during an inhalation segment; maintained in size during a breath holding segment; and reduced in size during an exhalation segment. Other suitable examples (e.g., color changing icons or the like) will be readily apparent to the skilled person.

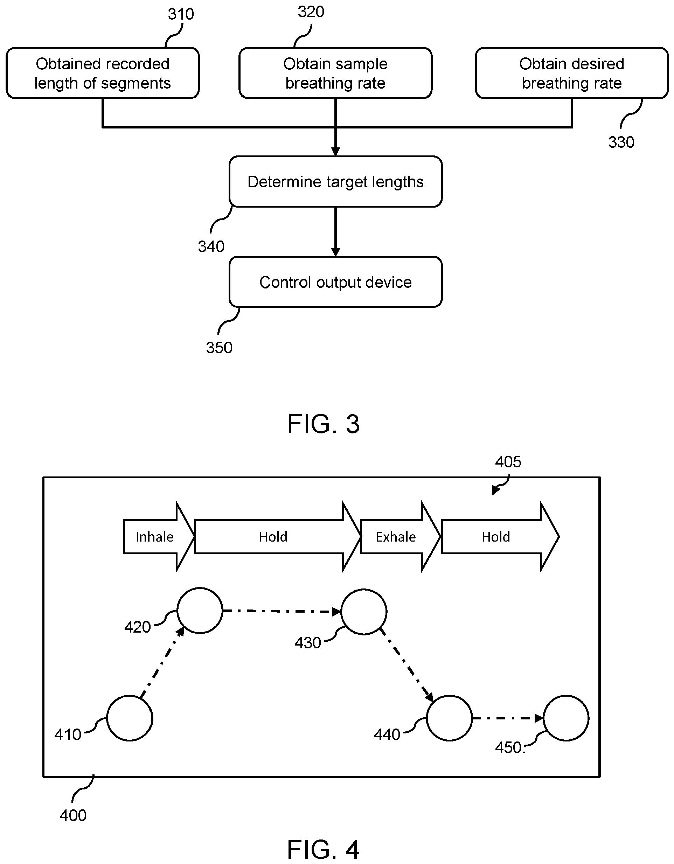

Fig. 3 is a flowchart that illustrates a method 300 for providing paced breathing guidance to an individual. The method 300 makes use of the proposed technique(s) in order to provide the paced breathing.

The method 300 comprises a step 310 of obtaining a recorded length for at least two different segments of a breathing cycle. Each recorded length is the monitored or determined length of a respective segment in a sample breathing cycle of the individual. A sample breathing cycle represents a historic or ground-truth breathing cycle of the individual (i.e., a breathing cycle that actually occurred).

It will be appreciated that the length of a segment may be defined as a measure of time, e.g., in seconds, milliseconds or using other suitable unit of time. An alternative label for a length of a segment is therefore a period.

The method 300 also comprises a step 320 of obtaining a sample breathing rate. A sample breathing rate represents a current, historic or previous breathing rate (e.g., in breaths per unit time) of the individual. The sample breathing cycle used to determine the recorded length may, for instance, be one (or an average) of the breathing cycles used in determining the sample breathing rate.

The method 300 also comprises a step 330 of obtaining a desired breathing rate. The desired breathing rate represents a target or desirable breathing rate for the individual, e.g., according to a particular paced breathing guidance protocol, a user-defined desired breathing rate or any other form of predetermined desired breathing rate. The precise mechanism by which a desired breathing rate is defined or obtained is immaterial to the underlying inventive concept.

The method 300 also comprises a step 340 of determining, for a plurality of (future/guided) breathing cycles, a target length for at least one of the at least two different segments of the breathing cycle. For the purposes of step 340, each of the plurality of breathing cycles is a different future or guided breathing cycle for the individual. The plurality of breathing cycles may be temporally adjacent to one another, e.g., to form a sequence or series of breathing cycles for paced breathing guidance. Thus, the plurality of breathing cycles may comprise a sequence or series of breathing cycles, more particularly a sequence or series of paced breathing cycles for paced breathing guidance.

Step 340 comprises processing the recorded lengths, the sample breathing rate and the desired breathing rate to determine the target length. In particular, each target length may be determined by processing at least the corresponding recorded length (for the same segment of a breathing cycle), the sample breathing rate and the desired breathing rate. The target length is proportional to the recorded length for said segment.

A number of example approaches for performing step 340 will be described later in this disclosure. Some described approaches share a same common recognition that the target length of a segment should be controlled to be proportional (i.e., calculated via multiplication) of the recorded length of the segment.

The method 300 also comprises a step 350 of controlling, for each of the plurality of (future/guided) breathing cycles, the output device to provide a user-perceptible output for guiding the individual to breathe, for said breathing cycle, in accordance with the target length for each of the at least two different segments.

Step 350 may comprise controlling the user-perceptible output through a sequence of different states or forms, each representing a different segment of the breathing cycle. The length during which the user-perceptible output is held in each state is controlled to be equal to the target length, to thereby provide paced breathing in accordance with the target length for each of the at least two different segments. Put another way, step 350 may change a user-perceptible output to follow a sequence of segments, each having target lengths. The state/form of a user-perceptible output should therefore differ between adjacent segments. Non-adjacent segments (e.g., breath holding segments separated by an inhalation/exhalation segment) may share a same user-perceptible output.

Specific suitable approaches for performing step 350 have been previously described and are well known in the art.

In some examples, the step 330 of obtaining a desired breathing rate comprises obtaining a same (i.e., single) desired breathing rate for each of the plurality of (future) breathing cycles.

In other examples, the step 330 of obtaining a desired breathing rate comprises obtaining a different desired breathing rate for each segment of each of the plurality of breathing cycles. In this way, the step 330 obtaining a desired breathing rate may comprise obtaining a desired breathing rate for each segment of each of the plurality of breathing cycles of the individual.

Accordingly, for each of the plurality of breathing cycles, the step of determining a target length for each segment may comprises processing the recorded length for said segment, the desired breathing rate for the breathing cycle of the individual, and the sample breathing rate for the individual. The target length for said segment will remain proportional to the recorded length for said segment.

The above approach allows, for instance, for the paced breathing guidance to gradually or incrementally change the guided breathing provided to the individual (in step 350). By way of example, the desired breathing rate may gradually reduce for successive breathing cycles, to encourage a gradual reduction in the breathing rate of the individual.

Thus, in one working example, the desired breathing rate for each of the plurality of breathing cycles initially starts at the sample breathing rate and gradually (i.e., sequentially with the plurality of breathing cycles) changes to a target breathing rate. A target breathing rate may, for instance, be an ultimate goal of the paced breathing guidance.

In one example, the plurality of breathing cycles comprises a sequence of no fewer than 10 breathing cycles, with the desired breathing rate for each breathing cycle sequentially decreasing through the sequence. The difference between the desired breathing rate of each breathing cycle in the sequence may be equal (for example) to a first value, being a difference between the sample breathing rate and the target breathing rate, divided by the total number of breathing cycles in the sequence. This provides an even and gradual decrease in the desired breathing rate output to the individual.

Other suitable patterns for changing a desired breathing rate of an individual could be used in other examples or embodiments, e.g., to follow a predetermined pattern or protocol. For instance, a desired breathing rate may reduce at a first speed for a first set/sequence of breathing cycles before reducing at a second speed for a second set/sequence of breathing cycles.

Using the proposed approach, each calculated time allocation for each segment will start from the beginning of next breath cycle. Thus, if the paced breath rate is to change while the breath cycle has not been finished, the previously calculated segment lengths will still be valid for the rest of the breath cycle.

Various approaches for performing step 340, for each breathing cycle, are hereafter described.

In a first approach, step 340 is performed by determining the target length of at least one segment of each of the plurality of breathing cycles by adjusting or defining the length of each segment proportionally to the ratio of the sample breathing rate and the desired breathing rate. More particularly, each segment may be controlled to be proportional to the recorded length for said segment multiplied by the ratio of the sample breathing rate and the desired breathing rate.

Thus, in one or more working examples, the target length $T_{TS}$ of at least one of, any and/or each given segment in a (future/guided) breathing cycle may be calculated using the following equation:

$$T_{TS} = \frac{R_S}{R_T} . T_{RS} \qquad (1)$$

where $R_S$ is the sample breathing rate; $R_T$ is the desired breathing rate for the breathing cycle (both of which are defined in breaths per minute) and $T_{RS}$ is the recorded length of the segment in the sample breathing cycle. As previously mentioned, the desired breathing rate may differ for different (future/guided) breathing cycles of the individual.

Equation (1) is functionally equivalent to the following equation:

$$T_{TS} = \frac{T_T}{T_R} . T_{RS} \qquad (2)$$

where $T_T$ is the total length of a target breathing cycle that achieves the desired breathing rate and $T_R$ is the total recorded length of the sample breathing cycle.

In a first approach, it is ensured that the proportion that each segment takes up in the plurality of (future) breathing cycles is the same as the proportion that each segment took up in the sample breathing cycle. This means that the breathing rhythm of the individual is encouraged or guided to remain substantially the same, which has been identified as improving compliance by an individual and ease of performing breathing.

In a second approach, step 340 is performed by setting the target length for each a first subset of one or segments to be the same as its corresponding recorded length and setting the target length for each other segment (not in the first subset) to be proportional to the ratio of the sample breathing rate and the desired breathing rate (i.e., proportional to $R_S \div R_T$). Thus, the target length of each segment in the first subset of one or more segments may be held at the recorded target length.

For instance, if present, the target length of each breath holding segment of a breathing cycle may be maintained at the recorded length of said breathing hold segment. Thus, the target length of each breath holding segment may be held at the recorded target length of said breath holding segment.

The target length of each other segment (e.g., any inhalation or exhalation segments) may be proportionally controlled, e.g., so that the ratio between any pair of other segments remains the same as during the sample breathing cycle. In this way, the target lengths of the segments, which are not part of the first subset of one or more segments, are controlled so that overall length of the breathing cycle achieves the desired breathing rate. Put another way, the target lengths of those segments not in the first subset of one or more segments contribute 100% of the modification(s) to target the desired breathing rate.

This can be achieved by applying, for each segment that is to be modified, the following equation to calculate its target length $T_{TS}$:

$$T_{TS} = \frac{60R_S - R_T.R_S.T_{HS}}{60.R_T - R_T.R_S.T_{HS}}.T_{RS} = \frac{R_S}{R_T}.\frac{60 - R_T.T_{HS}}{60 - R_S.T_{HS}}.T_{RS} \qquad (3)$$

where $T_{HS}$ is the sum of the recorded lengths for all segments that are to be held or maintained at its/their recorded length(s). For equation (3), the values $R_S$ and $R_T$ are defined in units of breaths per minute. The skilled person would be readily capable of adapting this equation for other units for breathing rate (e.g., breaths per second).

Equation (3) is functionally equivalent to the following equation:

$$T_{TS} = \frac{T_T - T_{HS}}{T_R - T_{HS}}.T_{RS} \qquad (4)$$

In a third approach, step 340 is performed by setting, for each of a plurality of subsets of one or more segments, the target length for each segment in that subset such that the segment contributes a particular percentage to the change in the total length of the breathing cycle to achieve the desired breathing rate. The target length of segments in a same subset may be maintained to be proportional to one another.

By way of example, the length of each a second subset of segments of a breathing cycle (e.g., breath holding segments) may be modified such that the second subset contributes X% (e.g., 20%) to the change of the total length of the breathing cycle to achieve the desired breathing rate, where as a third subset of segments of the breathing cycle (e.g., the inhalation and exhalation segments) may be modified such that the third subset contributes (100-X)% (e.g., 80%) to the change of the total length of the breathing cycle to achieve the desired breathing rate. In this way, the target length of each segment remains proportional to the recorded length of said segment, with different weights being applied to the segment dependent upon to which subset said segment belongs.

The value of X may be defined or determined by a user via a user interface, or may be predetermined based on pre-test results from several tested individuals. In some examples, the value of X is between 5 and 40, e.g., between 10 and 30, e.g., 20.

Thus, in a working example, the breath holding segments are adjusted slightly, whilst inhalation and exhalation segments will be adjusted heavily. For example, inhalation hold segment and exhalation hold segment may be adjusted by 20%, while inhalation and exhalation segments may be adjusted by 80%.

Additionally and/or alternatively to the approaches outlined above, the target length for each breath holding segment may be proportional to a weight that changes responsive to the sampled breathing rate. Thus, the target length for each breathing holding segment may be changed differently depending upon the sampled breathing rate. This, for instance, allows a different ratio to be used for breath holding segments when the sample breathing rate is lower compared to when the sample breathing rate is higher. In particular examples, a weight can be applied to (e.g., used to multiply) the ratio for breath holding segments to compensate the formula (for the first or third approaches).

A value of the weight is responsive to the sampled breathing rate, e.g., may switch between or have different values dependent upon the sampled breathing rate. Suitable values for the weight can be defined by pre-test of several test subjects or may be defined via a user interface. For instance, the pre-rest or user interface definition may define one or more ranges for the sampled breathing rate, each associated with a different weight to be applied to the ratio for the breath holding segments. Other suitable approaches for defining the relationship between a sampled breathing rate and a value of a weight will be apparent to the suitably skilled person.

In some examples, there may be a maximum allowable change to one or more of the segments of breathing. For instance, in some examples, a breath holding segment may have a maximum change of no more than 25%. As another example, a breath holding segment may have a maximum change of no more than 50%.

Several approaches for performing step 310, to obtain a recorded length of segments are envisaged, a non-exhaustive list of examples of which are hereafter provided.

In a simple example, a user or other individual may directly input or record the lengths of each segment. Thus, an individual, who is not necessarily the same individual for which paced breathing guidance is to be provided, can monitor the length of breathing segments of a sample breathing cycle (e.g., using a stopwatch or the like) and input the monitored lengths into a user interface to provide the recorded length of the segments.

In another example, an interactive user interface may be provided that enables an individual to mark points labelling the start and/or end points of different segments.

Fig. 4 conceptually illustrates one example of a display 400 of a user interface that allows an individual to record or mark the recorded lengths for each of a plurality of segments of a sample breathing cycle.

In this approach, a control program (such as a mobile application running on a mobile device with touchable display) provides or presents a display 400 at a user interface to the individual. The user interface may, for instance, provide a visual representation or display 400 of a normal breath 405 conceptually divided into different breath segments ("Inhale", "Hold", "Exhale", "Hold"). Each breath segment may be associated with two touch points 410, 420, 430, 440, 450 or interactive buttons, that identifying the start and/or end of the breath segment. For instance, an "Inhale" breath segment may be associated with a first and second touch point. Of course, adjacent breath segments may share a single touch point, e.g., to end one segment and start the next. The control program may guide or instruct the user to take a normal breath, and during breathing, the user will move through and touch the touch points of each breath segment as they breathe according to the breath segments. Thus, as a user inhales, they may move from the first touch point 410 to the second touch point 420. Subsequently, as the user holds their breath, they may move from the second touch point 420 to a third touch point 430. This is schematically illustrated in Fig. 4. An internal timer may monitor and record the times at which the individual touches a touch point one by one. With each recorded timestamp, the program will then be able calculate the time of each breath segment, i.e., the time difference between touching the two touch points associated with the breath segment.

An alternative approach to using a plurality of touch points for each segment is to simply provide an interactive user interface with which the user engages or interacts (e.g., presses and releases) to indicate the start/end of a breathing segment. For instance, a user may interact with the user interface to indicate the initiation of an inhale, before disengaging with the user interface to indicate a breath hold, before engaging again with the user interface to indicate an exhale, before disengaging again to indicate another breath hold, before engaging again to indicate an end to the breath hold.

Other variations and procedures for controlling a user interface to facilitate input of recorded lengths of different segments will be readily apparent to the skilled person.

Yet another approach makes use of a breath or respiratory sensor that directly monitors the breathing of the individual. Such a sensor would be able to monitor for different breath segments, e.g., by monitoring for movement or other characteristic changes that represent a movement through a breathing cycle.

Examples of breath sensors are well known in the art, such as those that directly monitor a (physical) movement of the chest or abdomen or those that monitor a visual motion of the chest/abdomen (e.g., using a camera). Yet other approaches monitor a fogging or defogging of a mask worn by the individual. Yet other approaches make use of microphones to monitor a sound of different breathing segments.

A wide variety of different respiratory monitoring systems are known in the art, any of which can be employed in the present invention. Some examples techniques are set out in Vanegas, Erik, Raul Igual, and Inmaculada Plaza. "Sensing systems for respiration monitoring: A technical systematic review." Sensors 20.18 (2020): 5446. Others will be readily apparent to the skilled person.

The sample breathing rate, for obtaining in step 220, can be trivially determined by multiplying 60 seconds with the reciprocal of the sum of all the recorded lengths for all segments of the sample breathing cycle. Alternatively, the sample breathing rate can be separately recorded or monitored, e.g., using standard respiratory rate monitoring mechanisms or techniques.

The skilled person would be readily capable of developing a control system for carrying out any herein described method in a system comprising an output device and the control system. Thus, each step of the flow chart may represent a different action performed by a control system, and may be performed by a respective module of the control system.

Embodiments may therefore make use of a control system. The control system can be implemented in numerous ways, with software and/or hardware, to perform the various functions required. A processor is one example of a control system which employs one or more microprocessors that may be programmed using software (e.g., microcode) to perform the required functions. A control system may however be implemented with or without employing a processor, and also may be implemented as a combination of dedicated hardware to perform some functions and a processor (e.g., one or more programmed microprocessors and associated circuitry) to perform other functions.

Examples of control system components that may be employed in various embodiments of the present disclosure include, but are not limited to, conventional microprocessors, application specific integrated circuits (ASICs), and field-programmable gate arrays (FPGAs).

In various implementations, a processor or control system may be associated with one or more storage media such as volatile and non-volatile computer memory such as RAM, PROM, EPROM, and EEPROM. The storage media may be encoded with one or more programs that, when executed on one or more processors and/or control systems, perform the required functions. Various storage media may be fixed within a processor or control system or may be transportable, such that the one or more programs stored thereon can be loaded into a processor or control system.

As previously explained, the output device is controllable to provide user-perceptible outputs that can be used to provide paced breathing guidance. More particularly, the control system may be configured to control the operation of the output device in order to controllably provide a user-perceptible output for providing paced breathing guidance in accordance with the target lengths of different segments of a breathing cycle.

It will be understood that disclosed methods are preferably computer-implemented methods. As such, there is also proposed the concept of a computer program comprising code means for implementing any described method when said program is run on a control system, such as a computer. Thus, different portions, lines or blocks of code of a computer program according to an embodiment may be executed by a control system or computer to perform any herein described method.

There is also proposed a non-transitory storage medium that stores or carries a computer program or computer code that, when executed by a control system, causes the control system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

**Claims**

1. A computer-implemented method (300) for controlling an output device (190) to provide paced breathing guidance to an individual, the computer-implemented method comprising:

   obtaining (310) a recorded length for each of at least two different segments (210, 220, 230, 240) in a sample breathing cycle (200) of the individual, each recorded length being the length of a respective segment in the sample breathing cycle (200);
   obtaining (320) a sample breathing rate of the individual;
   obtaining (330) a desired breathing rate for the individual; and
   determining (340), for a further breathing cycle of the individual, for at least one of the at least two different segments, a target length by processing the recorded length for said segment, the desired breathing rate for the individual, and the sample breathing rate for the individual,
   wherein the target length for said segment is proportional to the recorded length for said segment; and
   generating a control signal (350) for the output device (190) to provide a user-perceptible output for guiding the individual to breathe in accordance with the target length.

2. The computer-implemented method of claim 1, wherein the at least two different segments comprise at least one breath holding segment, and the at least one breath holding segment comprises a post-inhalation breath holding segment and a post-exhalation breath holding segment.

3. The computer-implemented method of claim 2, wherein, for each breath holding segment, the target length is the same as the recorded length for said segment of the sample breathing cycle.

4. The computer-implemented method of claim 2, wherein, for each breath holding segment, the difference between the target length and the recorded length is non-zero.

5. The computer-implemented method of claim 2, wherein, for each breath holding segment, the target length is proportional to the recorded length for said segment multiplied by a ratio of the sample breathing rate and the desired breathing rate.

6. The computer-implemented method of any of claims 4 or 5, wherein, for each breath holding segment, the target length is proportional to a weight that changes responsive to the sampled breathing rate.

7. The computer-implemented method of any of claims 2 to 6, wherein the at least two different segments comprise an inhalation segment and an exhalation segment.

8.   The computer-implemented method of claim 7, wherein, for the inhalation segment and the exhalation segment, the target lengths are proportional to the recorded length for said segment multiplied by a ratio of the sample breathing rate and the desired breathing rate.

9.   The computer-implemented method of claim 7 or 8, wherein, for the further breathing cycle:

a ratio between the target length of the inhalation segment and the recorded length of the inhalation segment is greater than a ratio between the target length of said breath holding segment and the recorded length of said breath holding segment; and/or
a ratio between the target length of the exhalation segment and the recorded length of the exhalation segment is greater than a ratio between the target length of said breath holding segment and the recorded length of said breath holding segment.

10.   The computer-implemented method of claim 8, when dependent upon any of claims 4 to 6, wherein, for the further breathing cycle:

a ratio between the target length of the inhalation segment and the recorded length of the inhalation segment is the same as a ratio between the target length of said breath holding segment and the recorded length of said breath holding segment; and/or
a ratio between the target length of the exhalation segment and the recorded length of the exhalation segment is the same as a ratio between the target length of said breath holding segment and the recorded length of said breath holding segment.

11.   The computer-implemented method of claim 9 or 10, comprising obtaining (310) a recorded length for all segments in the sample breathing cycle (200);

determining a target length for all segments in the sample breathing cycle (200),
wherein the sum of all target lengths, when defined in seconds, is equal to 60 times the reciprocal of the desired breathing rate, when defined in breaths per minute.

12.   The computer-implemented method of any of claims 1 to 11, wherein:

the step of obtaining a desired breathing rate comprises obtaining a desired breathing rate for each segment of each of a plurality of further breathing cycles of the individual; and
for each of the plurality of further breathing cycles, the step of determining a target length for each segment comprises processing the recorded length for said segment, the desired breathing rate for the breathing cycle of the individual, and the sample breathing rate for the individual, wherein the target length for said segment is proportional to the recorded length for said segment.

13.   The computer-implemented method of claim 12, wherein the desired breathing rate initially starts at the sample breathing rate and gradually changes to a target breathing rate over at least one of the plurality of further breathing cycles.

14.   A computer program product comprising computer program code means which, when executed on a computing device having a control system, cause the control system to perform all of the steps of the method according to any of claims 1 to 13.

15.   A system (100) for providing paced breathing guidance to an individual, the system comprising an output device (190) for providing user-perceptible outputs to an individual and a control system (110) configured to:

obtain (310) a recorded length for each of at least two different segments in a sample breathing cycle (200) of the individual, each recorded length being the length of a respective segment in a sample breathing cycle of the individual;
obtain (320) a sample breathing rate of the individual;
obtain (330) a desired breathing rate for the individual; and
determining (340), for a further breathing cycle of the individual, for a least one of the at least two different segments, a target length by processing the recorded length for said segment, the sample breathing rate and the desired breathing rate for the individual, wherein the target length for said segment is proportional to the recorded length for said segment; and

generate a control signal (350) for the output device (190) to provide a user-perceptible output for guiding the individual to breathe, for the breathing cycle, in accordance with the target length.

FIG. 1

FIG. 2

310

320

Obtained recorded
length of segments

Obtain sample
breathing rate

Obtain desired
breathing rate

330

Determine target lengths

340

Control output device

350

# FIG. 3

405

Inhale → Hold → Exhale → Hold

420

430

410

440

450

400

# FIG. 4

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/030278 A1 (KREMER KATHLEEN E [US] ET AL) 31 January 2019 (2019-01-31) | 1-8,10, 12-15 | INV. G16H20/30 |
| Y | * paragraph [0005] * <br> * paragraph [0119] * | 9 | G16H20/70 G16H10/60 A61M21/02 A63B23/18 |
| X | US 2021/330910 A1 (PERERA BODIYABADUGE DIMITHRI JOSEPH [AU] ET AL) 28 October 2021 (2021-10-28) <br> * paragraph [0147] – paragraph [0148] * <br> * paragraph [0151] – paragraph [0152] * <br> * figures 8, 11 * | 1,11-15 | ADD. A61M21/00 |
| X | US 2008/035147 A1 (KIRBY TODD [US] ET AL) 14 February 2008 (2008-02-14) <br> * paragraph [0003] * <br> * paragraph [0039] – paragraph [0040] * <br> * paragraph [0052] * <br> * paragraph [0070] * <br> * figure 2 * <br> * Equations (5), (6) * | 1,12-15 | |
| Y | WO 2019/240778 A1 (VARDAS SOLUTIONS LLC [US]) 19 December 2019 (2019-12-19) <br> * paragraph [0125] * | 9 | TECHNICAL FIELDS SEARCHED (IPC) <br><br> G16H A61M A63B |

The present search report has been drawn up for all claims

| Place of search | Date of completion of the search | Examiner |
|---|---|---|
| Munich | 27 October 2023 | Bonnet, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 478 372 A1

**ANNEX TO THE EUROPEAN SEARCH REPORT**
**ON EUROPEAN PATENT APPLICATION NO.**

EP 23 17 9491

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2023

| Patent document cited in search report | | Publication date | Patent family member(s) | | Publication date |
|---|---|---|---|---|---|
| US 2019030278 | A1 | 31-01-2019 | EP | 3658020 A1 | 03-06-2020 |
| | | | US | 2019030278 A1 | 31-01-2019 |
| | | | WO | 2019023427 A1 | 31-01-2019 |
| US 2021330910 | A1 | 28-10-2021 | US | 2021330910 A1 | 28-10-2021 |
| | | | WO | 2019157563 A1 | 22-08-2019 |
| US 2008035147 | A1 | 14-02-2008 | CN | 101528289 A | 09-09-2009 |
| | | | EP | 2066376 A2 | 10-06-2009 |
| | | | JP | 5345533 B2 | 20-11-2013 |
| | | | JP | 2010500117 A | 07-01-2010 |
| | | | US | 2008035147 A1 | 14-02-2008 |
| | | | WO | 2008021222 A2 | 21-02-2008 |
| WO 2019240778 | A1 | 19-12-2019 | NONE | | |

EPO FORM P0459

For more details about this annex : see Official Journal of the European Patent Office, No. 12/82